(19) Europäisches Patentamt
European Patent Office
Office européen des brevets

(11) Publication number: 0 006 254 B1

(12) **EUROPEAN PATENT SPECIFICATION**

(45) Date of publication of patent specification: 22.12.82

(21) Application number: 79200255.2

(22) Date of filing: 23.05.79

(51) Int. Cl.$^3$: A 01 N 35/10, A 01 N 37/12, C 07 C 131/00, C 07 D 317/58

(54) **Benzoin oxime derivatives, process for their preparation, compositions containing such derivatives and method for their use as fungicides.**

(30) Priority: 08.06.78 GB 2653078

(43) Date of publication of application: 09.01.80 Bulletin 80/1

(45) Publication of the grant of the patent: 22.12.82 Bulletin 82/51

(84) Designated Contracting States: AT CH DE FR GB IT LU

(56) References cited:
FR - A - 2 121 137
FR - A - 2 196 995
US - A - 2 963 399
US - A - 4 052 194

(73) Proprietor: SHELL INTERNATIONALE RESEARCH MAATSCHAPPIJ B.V.
Carel van Bylandtlaan 30
NL-2596 HR Den Haag (NL)

(72) Inventor: Ten Haken, Pieter
7, Bournemouth Drive
Herne Bay Kent (GB)
Inventor: Appleton, Robert Frederick
23, Laurel Avenue
Gravesend Kent (GB)
Inventor: Armitage, Brian Philip
14, Coombe Drive Snipeshill
Sittingbourne Kent (GB)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

Benzoin oxime derivatives, process for their preparation, compositions containing such derivatives and method for their use as fungicides.

The invention relates to fungicidal compositions which comprise as fungicidally active components one or more benzoin oxime derivative as hereinafter defined.

The invention further relates to the said fungicidally active components most of which are believed to be novel compounds.

Also the treatment of soil in which crops are growing or to be grown, which are subject to or are subjected to attack by fungi and the treatment of such crops or seeds thereof with a fungicidally effective amount of one or more compositions as claimed is included in the scope of the invention.

Certain aromatic oxime derivatives have been described previously as having fungicidal activity, and in some cases herbicidal activity. Thus French Patent Application No. 2,121,137 describes various 3-amino-, ureido- or thiureido- phenylketoxime derivatives having fungicidal properties against *Puccina recondita* and *Erysiphe cichoracearum* and United States Patent No. 2,963,399 describes poly-halogenated benzophenone oximes as having activity against *Alternaria aleracea, Sclerotina fruticola* and *Alternaria solani.* The present invention now discloses a different class of oxime derivatives which have fungicidal activity and accordingly the invention provides fungicidal compositions comprising as fungicidally active components one or more compound of the general formula

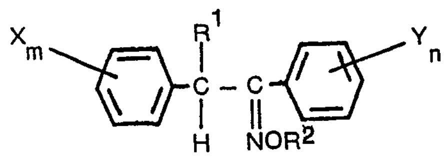

in which $R^1$ represents an acyloxy-, alkoxy-, alkylthio- or arylalkylthio- group, $R^2$ represents a hydrogen atom, an alkyl group with up to 4 carbon atoms, an optionally substituted aralkyl-, or an acyl group, X and Y independently represent halogen atoms, nitro, alkyl-, alkoxy-, alkoxycarbonyl- or methylene dioxy groups and m and n each are 0, 1 or 2; together with a carrier and/or a surface-active agent.

Compounds of the above formula are believed to be novel with the exception of those in which simultaneously $R^1$ is a methoxy or ethoxy group, $R^2$ is a hydrogen atom and m and n are zero.

Preferred compositions are those comprising compounds in which $R^1$ represents a methoxy- or an ethoxy group. If $R^1$ represents an acyloxy group, this group preferably is an acetoxy group. $R^2$ can suitably be a methyl- or ethyl group, but preferably represents a hydrogen atom or an acyl group, such as an acetyl group, a carbamyl- or a substituted carbamyl group, e.g. a methyl-carbamyl group. X and Y advantageously are halogen atoms, in particular chlorine or fluorine. Preferably m and n each are equal to 1.

Accordingly preferred compounds to be incorporated in fungicidal compositions include one or more of the following:

4′-chlorobenzoin-O-methylether-oxime
4′-chlorobenzoin-O-methylether-oxime
4,4′-difluorobenzoin-O-methylether-oxime
4-fluorobenzoin-O-methylether-oxime
acetyl-4-chlorobenzoin-O-methylether-oxime
4-methylbenzoin-O-methylether-oxime

Due to the presence of an asymmetric carbon atom in the compounds according to the invention, the above formula includes optical isomers.

Also geometric isomers (E and Z isomers) are encompassed by the formula in view of the fact that two different groups are bound to the C atom in the group $>C=NOR^2$. Whilst in many cases in particular the Z isomers exhibit a good fungicidal activity and accordingly preference is given to these isomers, it has been observed that in most instances the various isomers, both optical and geometric ones, as well as their mixtures are fully satisfactory and, accordingly, they are within the scope of the invention.

The term "carrier" as used herein means a material, which may be inorganic or organic and of synthetic or natural origin, with which the active compound is mixed or formulated to facilitate its application to the plant, seed, soil or other object to be treated, or its storage, transport or handling. The carrier may be a solid or a fluid. Any of the material usually applied in formulating fungicides may be used as carrier.

Suitable solid carriers are natural and synthetic clays and silicates for example natural silicas such as diatomaceous earths; magnesium silicates, for example, talcs; magnesium aluminium silicates, for example, attapulgites and vermiculites; aluminium silicates, for example, kaolinites, montmorillonites and micas; calcium carbonates; calcium sulphate; synthetic hydrated silicon oxides and synthetic calcium or aluminium silicates; elements such as for example, carbon and sulphur; natural and synthetic resins such as for example, coumarone resins, polyvinyl chloride and styrene polymers and copolymers; solid polychlorophenols; bitumen; waxes such as for example, beeswax, paraffin wax, and chlorinated mineral waxes; and solid fertilisers, for example superphosphates.

Examples of suitable fluid carriers are water, alcohols, such as for example, isopropanol, glycols; ketones such as for example, acetone, methyl ethyl ketone, methyl isobutyl ketone and cyclohexanone; ethers; aromatic hydrocarbons such as for example, benzene, toluene and xylene; petroleum fractions such as for example, kerosine, light mineral oils; chlorinated hydrocarbons, such as for example, carbon tetrachloride, perchloroethylene trichloroethane, including liquefied normally vaporous gaseous compounds. Mixtures of different liquids are often suitable.

The surface-active agent may be an emulsifying agent or a dispersing agent or a wetting agent; it may be nonionic or ionic. Any of the surface-active agents usually applied in formulating fungicides may be used. Examples of suitable surface-active agents are the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters or glycerol, sorbitan, sucrose or pentaerythritol; condensates of these with ethylene oxide and/or propylene oxide; condensation products of fatty alcohols or alkyl phenols for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example, sodium lauryl sulphate, sodium secondary alkyl sulphates, sodium salts or sulphonated castor oil, and sodium alkylaryl sulphonates such as sodium dodecylbenzene sulphonate; and polymers of ethylene oxide and copolymers of ethylene oxide and propylene oxide.

The compositions of the invention may be formulated as wettable powders, dusts, granules, solutions, emulsifiable concentrates, emulsions, suspension concentrates and aerosols, and will generally contain 0.5 to 95% w, preferably 0.5 to 75% w, of toxicant. Wettable powders are usually compounded to contain 25, 50, or 75% w of toxicant and usually contain, in addition to solid carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant and are diluted in the field with further solid carrier to give a composition usually containing 1/2—10% w of toxicant. Granules are usually prepared to have a size between 10 and 100 BS mesh (1.676—0.152 mm), and may be manufactured by agglomeration or impregnation techniques. Generally, granules will contain 1/2—25% w toxicant and 0—10% w of additives such as stabilisers, slow release modifiers and binding agents. Emulsifiable concentrates usually contain, in addition to the solvent and, when necessary, co-solvent, 20—50% w/v toxicant, 2—20% w/v emulsifiers and 0—20% w/v of appropriate additives such as stabilisers, penetrants and corrosion inhibitors. Suspension concentrates are compounded so as to obtain a stable, non-sedimenting, flowable product and usually contain 10—75% w toxicant, 0.5—15% w of dispersing agent, 0.1—10% w of suspending agents such as protective colloids and thixotropic agents, 0—10% w of appropriate additives such as defoamers, corrosion inhibitors, stabilisers, penetrants and stickers, and as carrier, water or an organic liquid in which the toxicant is substantially insoluble; certain organic solids or inorganic salts may be dissolved in the carrier to assist in preventing sedimentation or as anti-freeze agents for water.

The compositions of the invention may contain other ingredients, for example, protective colloids such as gelatin, glue, casein, gums, cellulose ethers, and polyvinyl alcohol; thixotropic agents, e.g. bentonites, sodium polyphosphates; stabilisers such as ethylene diamine tetraacetic acid, urea, triphenyl phosphate; other fungicides or pesticides; and stickers, for example non-volatile oils.

Aqueous dispersion and emulsions, for example, compositions obtained by diluting a wettable powder or an emulsifiable concentrate according to the invention with water, also lie within the scope of the present invention. The said emulsions may be of the water-in-oil or of the oil-in-water type, and may have a thick "mayonnaise"-like consistency.

For the preparation of the compounds of the above general formula different methods may be applied. According to a preferred method compounds of the formula A:

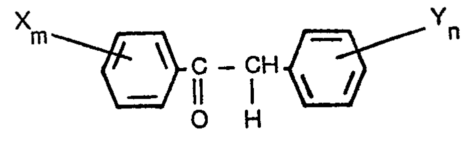

A

are first converted to compounds of the formula B:

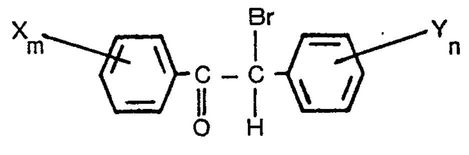

B

by reaction with bromine in the presence of a suitable solvent such as carbon tetrachloride and the like, under the influence of light radiation, and then, after reaction with alkoxide compounds, usually a

mixture of an alkalimetal alcoholate and the corresponding alcohol, converted by pyrolysis, preferably at a temperature in the range of 160—200°C, to form compounds of the formula C:

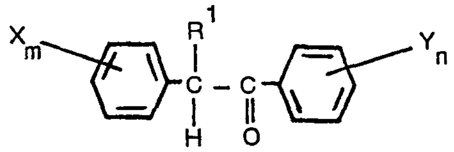

C

By reaction with hydroxylamine the oximes are obtained, from which further oxime derivatives may be prepared if so desired.

Alternatively the oximes may be prepared from the benzoin ethers which may be obtained by reaction of compounds of formula D:

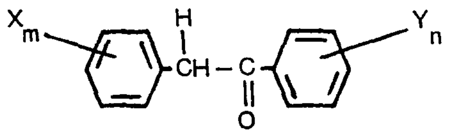

D

with bromine, in the manner as described above and then either by direct reaction with an alcohol or, after replacing the bromine atom by hydroxyl group, (suitably by a treatment with an alcoholic solution of an alkali metal alcoholate followed by a treatment with a diluted acid, suitably HCl), by conversion of the benzoins thus obtained with alkyliodide and silver oxide dissolved in an alkyl ester, such as ethylacetate. The benzoin ethers thus obtained are converted by subsequent reaction with hydroxylamine to the corresponding oximes.

The compounds of formula A and D may be conveniently prepared by a Grignard reaction or by a reaction catalysed by a Lewis acid such as $AlCl_3$.

The compound of formula C may also be prepared via benzoins by coupling of monoaromatic compounds by any suitable technique, such as benzoin condensation, e.g. by refluxing aromatic aldehydes in the presence of KCN and an alcohol or in the presence of a substituted thiazolium halide.

The invention is further illustrated by the following Examples.

## Example I

Preparation of 4-chlorobenzoin O-methylester (E)-oxime

(a) Dry benzyl chloride (50.6 g, 0.4 mole) in dry ether (200 cm³) reacted with magnesium (10.0 g, 0.41 g atom). When the reaction was complete, 4-chlorobenzamide (15.6 g, 0.1 mole) was added in portions with stirring. When the addition was complete the mixture was stirred and refluxed for 65 hours. The cooled mixture was then poured onto ice (400 g) containing concentrated sulphuric acid (40 g). Several extractions with ether yielded the crude product as a white solid (20.3 g, 88%). Recrystallization from methylated spirit gave white crystals of 4-chlorode-oxybenzoin m.pt. 106—106.5°C.

(b) 4-Chlorodeoxybenzoin (138 g, 0.6 mole) was suspended in carbon tetrachloride (800 cm³). A solution of bromine (96 g, 0.6 mole) in carbon tetrachloride was added dropwise while a strong light was shone on the reaction vessel to initiate the reaction. When the reaction was complete the solvent and dissolved gases were removed *in vacuo* to give a pale yellow solid (182 g, 98%). m.pt. 67—67.5°C.

(c) Bromo-4-chlorodeoxybenzoin (146.5 g, 0.475 mole) prepared as described under (b) was dissolved in abs. ethanol (800 cm³) at 30—35°C. This solution was added to a solution of sodium (38 g, 1.65 g atom) in abs. ethanol (750 cm³) and stirred overnight at room temperature. The mixture was poured into iced water (3 l) containing concentrated hydrochloric acid (350 cm³). The resulting pale yellow solid was filtered, washed with water and dried to yield 116 g (98%) m.pt. 89—90°C.

(d) 4-Chlorobenzoin (2.47 g, 0.01 mole) prepared as described under (c) was dissolved in dry ethyl acetate (10 cm³). To this solution was added freshly prepared, dry silver oxide (2.31 g, 0.01 mole) and dry methyl iodide (7.1 g, 0.05 mole). The stirred mixture was heated at 50°C for 3 hours. Filtration, followed by evaporation of solvents yielded a yellow oil (2.60 g, 100%). This product slowly solidified to give a yellow solid m.pt. 37—39°C.

(e) 4-Chlorobenzoin O-methyl ether (87.7 g, 0.337 mole) prepared as described under (d) was dissolved in ethanol (350 cm³) and treated with a solution of potassium hydroxide (112 g, 2.0 mole) and hydroxylamine hydrochloride (69.5 g, 1.0 mole) in water (400 cm³) at 25°C. After 2 hours, the mixture was poured into cold water (2 l). Extraction with ether yielded a brown oil which, upon trituration with petroleum ether, readily afforded the (E)-oxime (No. 12) as a pale yellow solid (67.5 g, 72%) m.pt. 103—105°C.

| Analysis: | | C | H | N |
|---|---|---|---|---|
| | Calculated for $C_{15}H_{14}NO_2Cl$ | 65.4 | 5.1 | 5.1% |
| | Found | 65.5 | 5.1 | 5.0 |
| | M.pt. 103—105°C | | | |

Example II

Preparation of 4-fluorobenzoin O-methylether (E) and (Z) oximes

(a) Phenylacetyl chloride (28.4 g, 0.184 mole) dissolved in fluorobenzene (130 cm³) and cooled in ice. This solution was added to freshly powdered aluminium chloride (27.0 g, 0.2 mole) with continued cooling and stirring. After the vigorous reaction subsided, the mixture was stirred at room temperature overnight and then poured onto ice (150 g) containing concentrated hydrochloric acid (50 cm³). Extraction afforded a pale brown solid (36.5 g, 88%).

Recrystallisation from petroleum ether gave a white solid m.pt. 80—81°C.

(b) The obtained 4-fluorodeoxybenzoin was converted to 4-fluorobenzoin O-methylether via bromo-4-fluorode-oxybenzoin and 4-fluorobenzoin in a manner entirely analogous as described in Example I under (b), (c) and (d).

(c) 4-Fluorobenzoin O-methyl ether (4.5 g, 0.0185 mole) was warmed at 50° for 3 hours with a mixture of potassium acetate (4.8 g, 0.049 mole) and hydroxylamine hydrochloride (3.0 g, 0.043 mole) in ethanol (80 cm³). After cooling and pouring into cold water, ether extraction resulted in the isolation of a yellow oil. The two oxime isomers contained therein were separated by silica gel column chromatography, eluting with methylene chloride. The Z-isomer No. 29 was recovered first as a pale yellow solid (1.0 g, 20%) m.pt. 93—97°C. Followed by the E-isomer No. 28 as a white solid (2.0 g, 41%) m.pt. 81—82°C.

| Analysis: | | C | H | N |
|---|---|---|---|---|
| (No. 28) | Calculated for $C_{15}H_{14}NO_2F$ | 69.5 | 5.4 | 5.4% |
| | Found | 69.3 | 5.4 | 5.4% |
| | M.pt. 81—82°C | | | |

| Analysis: | | C | H | N |
|---|---|---|---|---|
| (No. 29) | Calculated for $C_{15}H_{14}NO_2F$ | 69.5 | 5.4 | 5.4% |
| | Found | 69.7 | 5.6 | 5.2% |
| | M.pt. 93—97°C | | | |

Example III

Preparation of 2,4-dichlorobenzoin O-methylether (E) oxime

(a) 2,4-Dichlorophenylacetyl chloride (111 g, 0.5 mole) was added dropwise to a stirred mixture of freshly powdered aluminium chloride (78.5 g, 0.59 mole) in dry benzene (425 cm³). After the initial reaction had subsided, the mixture was heated at 70°C for 2 hours, cooled, then poured onto ice and extracted with methylene chloride. Evaporation of the solvent left a pale red solid. The colour was removed by washing with cold petroleum ether to give a white solid (123.5 g, 93%) m.pt. 94—97°C.

(b) 2′,4′-dichlorodeoxybenzoin (66.3 g, 0.25 mole) prepared as described under (a) was dissolved in carbon tetrachloride (250 cm³). To this was added bromine (40.0 g, 0.25 mole) dropwise while a strong light was shone on the reaction vessel to initiate the reaction. When the addition was complete, evaporation of the solvent afforded a pale brown oil (86.0 g, 100%).

(c) A slurry of bromo 2′,4′-dichlorodeoxybenzoin prepared as described under (b) (86.0 g, 0.25 mole) in methanol (50 cm³) was added to a solution of sodium (7.5 g, 0.33 g atom) in methanol (200 cm³) at 0°C. When the addition was complete the mixture was stirred at room temperature for 2 hours. After removal of excess solvent *in vacuo,* ether was added and the solution washed with water. The ether was evaporated from the dried solution *in vacuo* to leave a viscous oil. This oil was then heated at between 180°C and 200°C for 2 hours during which time a small amount of distillate was collected. The residue was the required product (71.1 g, 97%).

Slow solidification gave a solid m.pt. 33—35°C.

(d) By reaction with potassium hydroxide and hydroxylamine hydrochloride in a manner entirely analogous as described in Example I under (e), the 2,4-dichlorobenzoin O-methylether thus obtained was converted into the oxime (No. 46).

| Analysis: | | C | H | N |
|---|---|---|---|---|
| | Calculated for $C_{15}H_{13}NO_2Cl_2$ | 58.1 | 4.2 | 4.5% |
| | Found | 58.7 | 4.3 | 4.3% |
| | M.pt. 102—104°C | | | |

## Example IV

Preparation of acetyl 4-chlorobenzoin O-methylether (Z) oxime (No. 43)

4-Chlorobenzoin O-methylether (Z) oxime (No. 19) prepared in an analogous manner as described in Example I (3.4 g, 0.0123 Mole) was dissolved in dry methylene chloride (35 $cm^3$) and treated with acetyl chloride (1.0 g, 0.0127 mole) in the presence of triethylamine (2.5 $cm^3$). The mixture was stirred for 2 hours at room temperature, then poured into water; the organic layer was washed with water, dried and subsequently the solvent was evaporated *in vacuo* to yield the product (or acetyl derivative) as a pale brown oil (3.7 g, 95%).

| Analysis: | | C | H | N |
|---|---|---|---|---|
| (No. 43) | Calculated for $C_{17}H_{16}NO_3Cl$ | 64.4 | 5.1 | 4.4% |
| | Found | 64.7 | 5.3 | 4.2% |
| | Oil | | | |

| Analysis: | | C | H | N |
|---|---|---|---|---|
| (No. 19) | Calculated for $C_{15}H_{14}NO_2Cl$ | 65.4 | 5.1 | 5.1% |
| | Found | 65.4 | 4.9 | 4.7% |
| | M.pt. 108—111°C | | | |

## Example V

Preparation of 2'chloro-3,4-methylenedioxybenzoin O-methylether (E) oxime

(a) 2-Chlorobenzaldehyde (14.1 g, 0.1 mole) and piperonal (15.0 g, 0.1 mole) were dissolved in ethanol (50 $cm^3$) and refluxed for 2 hours with a solution of potassium cyanide (5.0 g) in water (10 $cm^3$). On refrigeration the product crystallised from the mixture and was filtered, washed with cold aqueous ethanol and dried to give a white solid (16.5 g, 57%) m.pt. 108—110°C.

(b) In an analogous manner as described in Example I under d and e the obtained 2'chloro-3,4-methylenedioxy benzoin was converted into the desired oxime. (no. 7).

| Analysis: | C | H | N |
|---|---|---|---|
| Calculated for $C_{16}H_{14}NO_4Cl$ | 60.0 | 4.4 | 4.4% |
| Found | 60.2 | 4.2 | 4.1% |
| M.pt. 108—109.5°C | | | |

## Example VI

Preparation of 4,4'-difluorobenzoin O-methylether oxime

(a) A mixture of 4-fluorobenzaldehyde (36.4 g, 0.294 mole), triethylamine (5.0 g) and 3-ethyl-5-(2-hydroxyethyl)-4-methyl thiazolium bromide catalyst (1.0 g) was refluxed overnight in ethanol (1000 $cm^3$). The mixture was cooled, poured into water and the crude product filtered. Recrystallisation from methylated spirit afforded white crystals (24.0 g, 66%) m.pt. 82—83°C.

(b) In an analogous manner as described in Example I under (d) and (e) the obtained 4,4'-difluorobenzoin was converted into the desired oxime (Nos. 26 and 27).

| Analysis: | | C | H | N |
|---|---|---|---|---|
| (No. 26) | Calculated for $C_{15}H_{13}NO_2F_2$ | 65.0 | 4.7 | 5.0% |
| | Found | 64.7 | 4.6 | 4.7% |
| | M.pt. 96—99°C | | | |

| Analysis: | | C | H | N |
|---|---|---|---|---|
| (No. 27) | Calculated for $C_{15}H_{13}NO_2F_2$ | 65.0 | 4.7 | 5.0% |
| | Found | 64.9 | 5.2 | 5.0% |
| | M.pt. 91—94°C | | | |

## Example VII

Following procedures similar to those described in the previous Examples a number of other oximes were prepared, physical properties of which are indicated in the Table below.

TABLE I

| Nr. | Compound | Analysis | | m.pt°C |
|---|---|---|---|---|
| 1. | benzoin O-methylether oxime | Calculated for $C_{15}H_{15}NO_2$: | C 74.7%; H 6.2%; N 5.8% | |
| | | Found: | 74.8 6.3 5.7 | Mpt 131—134 |
| 2. | benzoin O-ethylether oxime | Calculated for $C_{16}C_{17}NO_2$: | C 75.3%; H 6.7%; N 5.5% | |
| | | Found: | 75.4 6.3 5.3 | |
| 3. | 4-methoxybenzoin O-methylether oxime (E+Z) | Calculated for $C_{16}H_{17}NO_3$: | C 70.9%; H 6.3%; N 5.2% | |
| | | Found: | 70.8 6.3 4.8% | |
| 4. | 4,4′-dimethoxybenzoin O-methylether oxime (E+Z) | Calculated for $C_{17}H_{19}NO_4$: | C 67.9%; H 6.3%; N 4.6% | |
| | | Found: | 68.1 6.1 4.6 | Mpt 94—96 |
| 6. | 4′-chlorobenzoin O-methylether oxime (E) | Calculated for $C_{15}H_{14}NO_2Cl$: | C 65.4%; H 5.1%; N 5.1% | |
| | | Found: | 65.4 4.8 4.9 | Mpt 78—80 |
| 8. | 2,2′-dichlorobenzoin O-methylether oxime | Calculated for $C_{15}H_{13}NO_2Cl_2$: | C 58.1%; H 4.2%; N 4.5% | |
| | | Found: | 57.6 4.2 4.2 | Mpt 153—154 |
| 9. | 3,4,3′,4′-di(methylenedioxy-) benzoin O-methylether oxime | Calculated for $C_{17}H_{15}NO_6$: | C 62.0%; H 4.6%; N 4.2% | |
| | | Found: | 62.1 4.8 3.8 | Mpt 103—105 |
| 10. | 4′-chloro-3,4-methylenedioxybenzoin O-methylether oxime | Calculated for $C_{16}H_{14}NO_6Cl$: | C 60.0%; H 4.4%; N 4.4% | |
| | | Found: | 59.1 4.2 3.8 | Mpt 123 |
| 11. | 2′-chloro-4-methoxybenzoin O-methylether oxime | Calculated for $C_{16}H_{16}NO_3Cl$: | C 62.9%; H 5.3%; N 4.6% | |
| | | Found: | 62.6 5.3 4.6 | Mpt 112—115 |
| 13. | 4′-chlorobenzoin O-methylether oxime (Z) | Calculated for $C_{15}H_{14}NO_2Cl$: | C 65.4%; H 5.1%; N 5.1% | |
| | | Found: | 64.2 5.1 4.7 | Mpt 127—130 |
| 14. | 4,4′-dichlorobenzoin O-methylether oxime (Z) | Calculated for $C_{15}H_{13}NO_2Cl_2$: | C 58.1%; H 4.2%; N 4.5% | |
| | | Found: | 58.1 4.2 4.0 | Mpt 99—100 |
| 15. | 4,4′-dichlorobenzoin O-methylether oxime (E) | Calculated for $C_{15}H_{13}NO_2Cl_2$: | C 58.1%; H 4.2%; N 4.5% | |
| | | Found: | 58.5 4.2 4.3 | Mpt 138—140 |
| 16. | methyl 2′-chloro-4-methoxybenzoin O-methylether | Calculated for $C_{17}H_{18}NO_3Cl$: | C 63.9%; H 5.6%; N 4.4% | |
| | | Found: | 64.4 6.1 3.8 | Oil |
| 17. | methyl 4′-chlorobenzoin O-methyl-ether oxime (E) | Calculated for $C_{16}H_{16}NO_2Cl$: | C 66.3%; H 5.5%; N 4.8% | |
| | | Found: | 66.6 6.1 5.0 | Oil |
| 18. | methyl 4-chlorobenzoin O-methylether oxime (E) | Calculated for $C_{16}H_{16}NO_2Cl$: | C 66.3%; H 5.5%; N 4.8% | |
| | | Found: | 66.3 5.9 4.7 | |
| 20. | methyl 4-chlorobenzoin O-methylether oxime (Z) | Calculated for $C_{16}H_{16}NO_2Cl$: | C 66.3%; H 5.5%; N 4.8% | |
| | | Found: | 66.6 5.7 5.1 | Mpt 74—76 |

TABLE I cont'd

| Nr. | Compound | Analysis | m.pt.°C |
|---|---|---|---|
| 21. | methylcarbamyl 4-chlorobenzoin O-methylether oxime (E) | Calculated for $C_{17}H_{17}N_2O_3Cl$:<br>Found: | C 61.4%; H 5.1%; N 8.4%<br>56.8 4.8 7.7 |
| 22. | 4′-chloro-4-methoxybenzoin O-methylether oxime | Calculated for $C_{16}H_{16}NO_3Cl$:<br>Found: | C 63.0%; H 5.3%; N 4.6%<br>62.9 5.6 4.8<br>Mpt 106—109 |
| 23. | 4′-chloro-4-methoxybenzoin O-methylether oxime (Z) | Calculated for $C_{16}H_{16}NO_3Cl$:<br>Found: | C 63.0%; H 5.3%; N 4.6%<br>62.8 5.6 4.6<br>Oil |
| 24. | 4-chloro-4′-methoxybenzoin O-methylether oxime (E) | Calculated for $C_{16}H_{16}NO_3Cl$:<br>Found: | C 63.0%; H 5.3%; N 4.6%<br>62.2 5.3 4.6<br>Mpt 123 |
| 25. | 4-chloro-4′-methoxybenzoin O-methylether oxime (Z) | Calculated for $C_{16}H_{16}NO_3Cl$:<br>Found: | C 63.0%; H 5.3%; N 4.6%<br>62.8 5.3 4.4<br>Oil |
| 30. | 4-chlorobenzoin O-ethylether oxime (E) | Calculated for $C_{16}H_{16}NO_2Cl$:<br>Found: | C 66.4%; H 5.5%; N 4.8%<br>66.7 5.7 4.6<br>Mpt 110—113 |
| 31. | 4-chlorobenzoin O-ethylether oxime (Z) | Calculated for $C_{16}H_{16}NO_2Cl$:<br>Found: | C 66.4%; H 5.5%; N 4.8%<br>66.6 5.9 4.8<br>Oil |
| 32. | acetyl 4-chlorobenzoin O-methyl-ether oxime (E) | Calculated for $C_{17}H_{16}NO_3Cl$:<br>Found: | C 64.4%; H 5.1%; N 4.4%<br>62.8 5.6 4.4<br>Oil |
| 33. | benzyl 4-chlorobenzoin O-methylether oxime (E) | Calculated for $C_{22}H_{20}NO_2Cl$:<br>Found: | C 72.3%; H 5.5%; N 3.8%<br>71.9 5.5 3.9<br>Oil |
| 34. | m-phenoxybenzyl 4-chlorobenzoin O-methylether oxime | Calculated for $C_{28}H_{24}NO_3Cl$:<br>Found: | C 73.4%; H 5.2%; N 3.0%<br>73.4 5.3 2.9<br>$n_D^{23} = 1.6065$ |
| 35. | methyl 4-chlorobenzoin O-ethyl-ether oxime (E) | Calculated for $C_{17}H_{18}NO_2Cl$:<br>Found: | C 67.2%; H 5.9%; N 4.6%<br>67.3 5.8 4.3<br>Oil |
| 36. | methyl 4-chlorobenzoin O-ethyl-ether oxime (Z) | Calculated for $C_{17}H_{18}NO_2Cl$:<br>Found: | C 67.2%; H 5.9%; N 4.6%<br>67.6 6.2 4.8<br>Oil |
| 37. | 4-chlorobenzoin O-acetyl-ester oxime (E) | Calculated for $C_{16}H_{14}NO_3Cl$:<br>Found: | C 63.4%; H 4.6%; N 4.6%<br>63.2 4.2 5.1<br>Mpt 122—124 |
| 38. | 4-chlorobenzoin O-acetyl ester oxime (Z) | Calculated for $C_{16}H_{14}NO_3Cl$:<br>Found: | C 63.4%; H 4.6%; N 4.6%<br>64.2 4.6 5.1<br>Mpt 90—93 |
| 39. | acetyl 4-chlorobenzoin O-acetyl-ester oxime (E) | Calculated for $C_{18}H_{16}NO_4Cl$:<br>Found: | C 62.5%; H 4.6%; N 4.0%<br>60.2 4.5 3.7<br>Oil |

TABLE I cont'd

| Nr. | Compound | Analysis | m.pt°C |
|---|---|---|---|
| 40. | acetyl 4-chlorobenzoin 0-acetylester oxime (Z) | Calculated for $C_{18}H_{16}NO_4Cl$:<br>Found: | C 62.5%; H 4.6%; N 4.0%<br>63.3 4.7 3.9<br>Oil |
| 41. | benzoyl 4-chlorobenzoin 0-methylether oxime (E) | Calculated for $C_{22}H_{18}NO_3Cl$:<br>Found: | C 69.5%; H 4.8%; N 3.7<br>69.4 4.8 3.7<br>Mpt 88—89 |
| 42. | benzoyl 4-chlorobenzoin 0-methylether oxime (Z) | Calculated for $C_{22}H_{18}NO_3Cl$:<br>Found: | C 69.5%; H 4.8%; N 3.7%<br>69.1 4.9 3.4<br>Oil |
| 44. | 4,4'-dimethylbenzoin 0-methylether oxime (E) | Calculated for $C_{17}H_{19}NO_2$:<br>Found: | C 75.8%; H 7.1%; N 5.2%<br>75.8 7.1 4.9<br>Mpt 147—153 |
| 45. | 4,4'-dimethylbenzoin 0-methylether oxime (Z) | Calculated for $C_{17}H_{19}NO_2$:<br>Found: | C 75.8%; H 7.1%; N 5.2%<br>75.2 7.2 4.7<br>Mpt 119—122 |
| 47. | 2,4-dichlorobenzoin-0-methylether oxime (E) | Calculated for $C_{15}H_{13}NO_2Cl_2$:<br>Found: | C 58.1%; H 4.2%; N 4.5%<br>58.7 4.3 4.3<br>Mpt 102—104 |
| 48. | acetyl-2,4-dichlorobenzoin-0-methylether oxime (E) | Calculated for $C_{17}H_{15}NO_3Cl_2$:<br>Found: | C 57.9%; H 4.3%; N 4.0%<br>58.0 4.4 4.0<br>Oil |
| 49. | 3-chlorobenzoin-0-methylether oxime (E) | Calculated for $C_{15}H_{14}NO_2Cl$:<br>Found: | C 65.4%; H 5.1%; N 5.1%<br>65.4 5.2 4.7<br>Mpt 94.5—95.5 |
| 50. | 3-chlorobenzoin-0-methylether oxime (Z) | Calculated for $C_{15}H_{14}NO_2Cl$:<br>Found: | C 65.4%; H 5.1%; N 5.1%<br>65.4 5.0 4.5<br>Mpt 87—90 |
| 51. | acetyl-4-fluorobenzoin-0-methylether oxime (Z) | Calculated for $C_{17}H_{16}NO_3F$:<br>Found: | C 67.8%; H 5.3%; N 4.6%<br>67.8 5.4 4.3<br>Oil |
| 52. | acetyl-3-chlorobenzoin-0-methylether oxime (E) | Calculated for $C_{17}H_{16}NO_3Cl$:<br>Found: | C 64.2%; H 5.0%; N 4.4%<br>64.4 5.3 4.3<br>Oil |
| 53. | acetyl-3-chlorobenzoin-0-methylether oxime (Z) | Calculated for $C_{17}H_{16}NO_3Cl$:<br>Found: | C 64.2%; H 5.0%; N 4.4%<br>64.5 5.4 4.4<br>Oil |
| 54. | 2,4-dichlorobenzoin-0-methylether oxime (Z) | Calculated for $C_{15}H_{13}NO_2Cl_2$:<br>Found: | C 58.1%; H 4.2%; N 4.5%<br>56.8 4.5 4.7<br>Oil |
| 55. | 4'-nitrobenzoin-0-methylether oxime (E) | Calculated for $C_{15}H_{14}N_2O_4$:<br>Found: | C 62.9%; H 4.9%; N 9.8%<br>62.6 4.9 9.6<br>Mpt 179—183 |
| 56. | 4'-nitrobenzoin-0-methyl ether oxime (Z) | Calculated for $C_{15}H_{14}N_2O_4$:<br>Found: | C 62.9%; H 4.9%; N 9.8%<br>62.5 4.9 9.6<br>Mpt 186—188 |

TABLE I cont'd

| Nr. | Compound | Analysis | m.pt°C |
|---|---|---|---|
| 57. | 4-bromobenzoin-O-methylether-oxime (E) | Calculated for $C_{15}H_{14}NO_2Br$:<br>Found: | C 56.2%; H 4.4%; N 4.4%<br>56.2 4.4 4.5<br>Mpt 101—103 |
| 58. | 4-bromobenzoin-O-methylether-oxime (Z) | Calculated for $C_{15}H_{14}NO_2Br$:<br>Found: | C 56.2%; H 4.4%; N 4.4%<br>56.2 4.4 4.1<br>Mpt 114—117 |
| 59. | benzoin-S-methylthioether oxime (Z) | Calculated for $C_{15}H_{15}NOS$:<br>Found: | C 70.0%; H 5.5%; N 5.4%<br>70.6 5.9 5.4<br>Mpt 104—105 |
| 60. | benzoin-S-methylthioether oxime (E) | Calculated for $C_{15}H_{15}NOS$:<br>Found: | C 70.0%; H 5.5%; N 5.4%<br>70.1 6.1 5.3<br>Mpt 157—158 |
| 61. | 4-methylbenzoin-O-methyl-ether oxime (E) | Calculated for $C_{16}H_{17}NO_2$:<br>Found: | C 74.0%; H 7.0%; N 5.8%<br>74.7 6.8 5.3<br>Mpt 115—118 |
| 62. | 4-methylbenzoin-O-methyl-ether oxime (Z) | Calculated for $C_{16}H_{17}NO_2$:<br>Found: | C 74.0% H7.0%; N 5.8%<br>75.7 7.0 5.4<br>Mpt 103—105 |
| 63. | 4,4'-di(methoxycarbonyl)-ben-zoin-O-methylether oxime (E) | Calculated for $C_{19}H_{19}NO_6$:<br>Found: | C 64.0%; H 5.3%; N 3.9%<br>63.9 5.4 3.8<br>Mpt 140—141 |
| 64. | 4,4'-di(methoxycarbonyl)ben-zoin-O-methylether oxime (Z) | Calculated for $C_{19}H_{19}NO_6$:<br>Found: | C 64.0%; H 5.3%; N 3.9%<br>64.1 5.4 3.8<br>Mpt 125—128 |
| 65. | 2 chlorobenzoin-O-methyl-ether oxime (E) | Calculated for $C_{15}H_{14}NO_2Cl$:<br>Found: | C 65.3%; H 5.1%; N 5.1%<br>65.4 5.1 5.0<br>Mpt 88—92 |
| 66. | benzoin-S-benzylthioether oxime (E+Z) | Calculated for $C_{21}H_{19}NOS$:<br>Found: | C 75.9%; H 5.4%; N 4.2%<br>75.8 5.8 4.2 |
| 67. | 4'-methylbenzoin-O-methyl-ether oxime (E) | Calculated for $C_{16}H_{17}NO_2$:<br>Found: | C 74.4%; H 6.6%; N 5.8%<br>74.4 6.6 5.2<br>Mpt 151—153 |
| 68. | 4'-methylbenzoin-O-methyl-ether oxime (Z) | Calculated for $C_{16}H_{17}NO_2$:<br>Found: | C 74.4%; H 6.6%; N 5.8%<br>75.1 7.0 5.5<br>Mpt 86—89 |
| 69. | 3,4,3',4'-di(methylenedioxy)-benzoin-O-methylether oxime (Z) | Calculated for $C_{17}H_{15}NO_6$:<br>Found: | C 62.0%; H 4.6%; N 4.3%<br>61.4 4.5 3.9<br>Mpt 130—133 |
| 70. | 4-chlorobenzoin-S-methylthio-ether oxime (E) | Calculated for $C_{15}H_{14}NOSCl$:<br>Found: | C 61.8%; H 4.8%; N 4.8%<br>62.0 4.8 4.7<br>Mpt 127—129 |
| 71. | 4-chlorobenzoin-S-methylthio-ether oxime (Z) | Calculated for $C_{15}H_{14}NOSCl$:<br>Found: | C 61.8%; H 4.8%; N 4.8%<br>62.2 4.9 4.6<br>Mpt 105—106 |

All structures, configurations and purities are supported by NMR spectroscopic data.

Example VIII

Fungicidal activity

The fungicidal activity of the compounds according to the invention was investigated by the following foliar spray tests:

(1) Activity against vine downy mildew (Plasmopara viticola Pv.a)

The test is a direct antisporulant one using a foliar spray. The lower surfaces of leaves of whole vine plants, are inoculated by spraying with an aqueous suspension containing $10^5$ zoosporangia/ml 4 days prior to treatment with the test compound. The inoculated plants are kept for 24 hours in a high humidity compartment, 48 hours at glasshouse ambient temperature and humidity and then returned for a further 24 hours to high humidity. The plants are then dried and infected leaves detached and sprayed on the lower surfaces at a dosage of 1 kilogram of active material per hectare using a track sprayer. After drying the petioles of the sprayed leaves are dipped in water and the leaves returned to high humidity for a further 72 hours incubation, followed by assessment. Assessment is based on the percentage of the leaf area covered by sporulation compared with that on control leaves.

2) Activity against vine downy mildew (Plasmopara viticola Pv.t)

The test is a translaminar protectant one using a foliar spray. The upper surfaces of leaves of whole vine plants are sprayed at a dosage of 1 kilogram of active material per hectare using a track sprayer. The lower surfaces of the leaves are then inoculated, up to 6 hours after treatment with the test compound, by spraying with an aqueous suspension containing $10^5$ zoosporangia/ml. The inoculated plants are kept for 24 hours in a high humidity compartment, 4 days at glasshouse ambient temperature and humidity and then returned for a further 24 hours to high humidity. Assessment is based on the percentage of the leaf area covered by sporulation compared with that on control leaves.

3) Activity against barley powdery mildew (Erysiphe graminis Eg.)

The test measures the direct antisporulant activity of compounds applied as a foliar spray. For each compound about 40 barley seedlings were grown to the one-leaf stage in a plastic pot of sterile potting compost. Inoculation was effected by dusting the leaves with conidia of *Erysiphe graminis, spp. hordei.* 24 hours after inoculation the seedlings were sprayed with a solution of the compound in a mixture of acetone (50%), surfactant (0.04%) and water using a track sprayer. The rate of application was equivalent to 1 kg of active material per hectare. First assessment of disease was made 5 days after treatment, when the overall level of sporulation on the treated pots were compared with that on control pots.

4) Activity against potato late blight (Phytophthora infestans Pi.p)

The test measures the direct protectant activity of compounds applied as a foliar spary. Tomato plants, cultivar Ailsa Craig, 1—15 cm high, in monopots are used. The whole plant is sprayed at a dosage of 1 kilogram of active material per hectare using a track sprayer. The plant is then inoculated up to 6 hours after treatment with the test compound, by spraying with an aqueous suspension containing $5 \times 10^3$ zoosporangia/ml. The inoculated plants are kept in high humidity for 3 days. Assessment is based on a comparison between the levels of disease on the treated and control plants.

The extent of disease control is expressed as a control rating according to the criteria:—

0 = less than 50% disease control
1 = 50—80% disease control
2 = greater than 80% disease control

In the following Table the disease control ratings are given for the compounds described in the previous Examples.

TABLE II

| No. | Pv.a | Pv.t | Eg. | Pi.p |
|---|---|---|---|---|
| 1 | 1 | 0 | 1 | 1 |
| 2 | 0 | 0 | 0 | 0 |
| 3 | 1 | 0 | 0 | 0 |
| 4 | 0 | 0 | 0 | 0 |
| 6 | 2 | 2 | 1 | 2 |
| 7 | 0 | 0 | 1 | 2 |

TABLE II cont'd

| No. | Pv.a | Pv.t | Eg. | Pi.p |
|---|---|---|---|---|
| 8 | 0 | 0 | 0 | 0 |
| 9 | 0 | 0 | 0 | 1 |
| 10 | 0 | 0 | 0 | 1 |
| 11 | 0 | 0 | 0 | 0 |
| 12 | 2 | 2 | 0 | 2 |
| 13 | 0 | 1 | 0 | 2 |
| 14 | 0 | 2 | 0 | 1 |
| 15 | 2 | 0 | 0 | 2 |
| 16 | 0 | 0 | 0 | 0 |
| 17 | 0 | 0 | 0 | 0 |
| 18 | 0 | 0 | 2 | 0 |
| 19 | 2 | 2 | 2 | 0 |
| 20 | 2 | 2 | 0 | 0 |
| 21 | 0 | 0 | 0 | 0 |
| 22 | 0 | 0 | 0 | 1 |
| 23 | 0 | 0 | 0 | 1 |
| 24 | 0 | 0 | 0 | 1 |
| 25 | 0 | 2 | 0 | 1 |
| 26 | 1 | 2 | 0 | 1 |
| 27 | 2 | 2 | 0 | 2 |
| 28 | 2 | 2 | 0 | 2 |
| 29 | 2 | 2 | 0 | 2 |
| 30 | 0 | 2 | 0 | 1 |
| 31 | 2 | 2 | 0 | 2 |
| 32 | 2 | 2 | 1 | 0 |
| 33 | 0 | 0 | 0 | 0 |
| 34 | 0 | 0 | 0 | 0 |
| 35 | 0 | 2 | 0 | 0 |
| 36 | 0 | 1 | 0 | 1 |
| 37 | 0 | 0 | 0 | 0 |
| 38 | 0 | 2 | 0 | 0 |

TABLE II cont'd

| No. | Pv.a | Pv.t | Eg. | Pi.p |
|---|---|---|---|---|
| 39 | 0 | 0 | 0 | 0 |
| 40 | 0 | 1 | 0 | 0 |
| 41 | 0 | 0 | 0 | 0 |
| 42 | 0 | 2 | 0 | 0 |
| 43 | 2 | 2 | 2 | 2 |
| 44 | 1 | 0 | 2 | 2 |
| 45 | 2 | 2 | 2 | 2 |
| 46 | 0 | 1 | 0 | 0 |
| 47 | 0 | 1 | 0 | 0 |
| 48 | 0 | 0 | 2 | 1 |
| 49 | 2 | 2 | 1 | 2 |
| 50 | 2 | 2 | 2 | 2 |
| 51 | 2 | 2 | 2 | 2 |
| 52 | 1 | 1 | 1 | 1 |
| 53 | 2 | 2 | 2 | 2 |
| 54 | 0 | 0 | 0 | 1 |
| 55 | 0 | 0 | 0 | 0 |
| 56 | 0 | 1 | 0 | 0 |
| 57 | 1 | 1 | 0 | 2 |
| 58 | 2 | 2 | 1 | 2 |
| 59 | 0 | 0 | 0 | 2 |
| 60 | 0 | 0 | 0 | 0 |
| 61 | 2 | 2 | 0 | 1 |
| 62 | 2 | 2 | 1 | 2 |
| 63 | 0 | 0 | 0 | 0 |
| 64 | 0 | 2 | 0 | 1 |
| 65 | 0 | 0 | 0 | 0 |
| 66 | 0 | 0 | 0 | 2 |
| 67 | 0 | 0 | 0 | 0 |
| 68 | 2 | 0 | 0 | 2 |
| 69 | 2 | 0 | 0 | 0 |

TABLE II cont'd

| No. | Pv.a | Pv.t | Eg. | Pi.p |
|---|---|---|---|---|
| 70 | 0 | 0 | 0 | 0 |
| 71 | 2 | 2 | 0 | 1 |

**Claims for the Contracting States: CH DE FR GB IT LU**

1. Fungicidal compositions comprising as fungicidally active components one or more compounds of the general formula

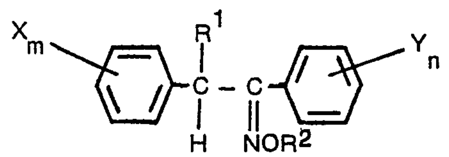

in which $R^1$ represents an acyloxy-, alkoxy-, alkylthio- or arylalkylthio group, $R^2$ represents a hydrogen atom, an alkyl group with up to 4 carbon atoms, an optionally substituted aralkyl- or an acyl group, X and Y independently represent halogen atoms, nitro-, alkyl-, alkoxy-, alkoxycarbonyl-, or methylenedioxy groups and m and n each are 0, 1 or 2, together with a carrier and/or a surface-active agent.

2. Fungicidal compositions as claimed in claim 1 in which $R^1$ represents a methoxy- or ethoxy group.

3. Fungicidal compositions as claimed in claim 1 or 2 in which $R^2$ represents a hydrogen atom or an acyl group.

4. Fungicidal compositions as claimed in any one of claims 1 to 3, in which X and/or Y represents a halogen atom.

5. Fungicidally active compounds of the formula

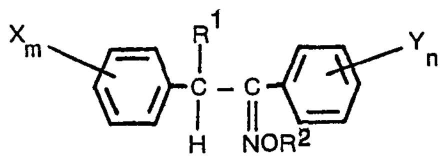

in which $R^1$, $R^2$, X, Y, m and n have the aforesaid meaning with the proviso that if $R^1$ represents alkoxy and $R^2$ is a hydrogen atom, m and n should not both be equal to 0.

6. 4 Chlorobenzoin O-methylether oxime.

7. 4-Methylbenzoin O-methylether oxime.

8. 4'-Chlorobenzoin O-methylether oxime.

9. 4,4'-difluorobenzoin O-methylether oxime.

10. 4 Fluorobenzoin O-methylether oxime.

11. Acetyl 4-chlorobenzoin O-methylether oxime.

12. Process for the preparation of compounds of the general formula of claim 1, in which compounds of the formula

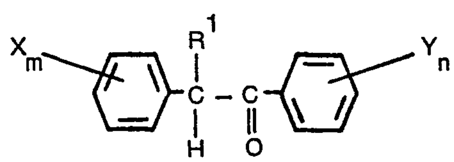

wherein $R^1$ represents an acyloxy- or alkoxy group are reacted with hydroxylamine optionally followed by reacting the obtained oximes with a compound $R^2Z$, in which Z is halide to form the corresponding oxime derivatives.

13. Process as claimed in claim 12, in which compounds of the formula

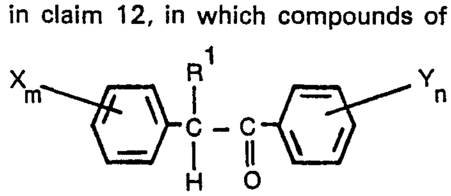

are applied which have been obtained by reaction of compounds of the formula

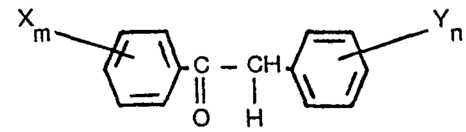

with bromine in the presence of a solvent under the influence of light radiation, followed by reaction with an alkoxide compound and pyrolysis.

14. Process as claimed in claim 13, in which the pyrolysis is carried out at a temperature in the range of 160—200°C.

15. Method of protecting crops from attack by fungi in which crops subject to or subjected to such attack, seeds of such crops and/or soil in which the said crops are growing or to be grown, are treated with a fungicidally effective amount of one or more compounds of the general formula of claim 1.

**Claims for the Contracting State: AT**

1. Fungicidal compositions comprising as fungicidally active components one or more compounds of the general formula

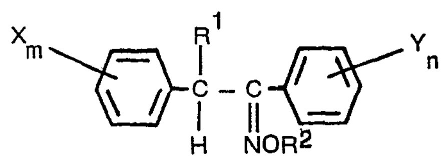

in which $R^1$ represents an acyloxy-, alkoxy-, alkylthio- or arylalkylthio group, $R^2$ represents a hydrogen atom, an alkyl group with up to 4 carbon atoms, an optionally substituted aralkyl- or an acyl group, X and Y independently represent halogen atoms, nitro-, alkyl-, alkoxy-, alkoxycarbonyl-, or methylenedioxy groups and m and n each are 0, 1 or 2, together with a carrier and/or a surface-active agent.

2. Fungicidal compositions as claimed in claim 1 in which $R^1$ represents a methoxy- or ethoxy group.

3. Fungicidal compositions as claimed in claim 1 or 2 in which $R^2$ represents a hydrogen atom or an acyl group.

4. Fungicidal compositions as claimed in any one of claims 1 to 3, in which X and/or Y represents a halogen atom.

5. Fungicidally compositions as claimed in claim 1 in which the fungicidally active component is 4-chlorobenzoin O-methylether oxime; 4-methylbenzoin O-methylether oxime; 4′-chlorobenzoin O-methylether oxime; 4,4′-difluorobenzoin O-methylether oxime; 4-fluorobenzoin O-methylether oxime; or acetyl 4-chlorobenzoin O-methylether oxime.

6. Process for the preparation of compounds of the general formula of claim 1, in which compounds of the formula

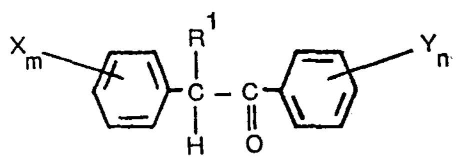

wherein $R^1$ represents an acyloxy- or alkoxy group are reacted with hydroxylamine optionally followed by reacting the obtained oximes with a compound $R^2Z$, in which Z is halide to form the corresponding oxime derivatives.

7. Process as claimed in claim 6, in which compounds of the formula

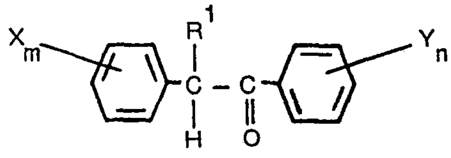

are applied which have been obtained by reaction of compounds of the formula

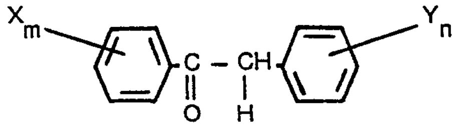

with bromine in the presence of a solvent under the influence of light radiation, followed by reaction with an alkoxide compound and pyrolysis.

8. Process as claimed in claim 7, in which the pyrolysis is carried out at a temperature in the range of 160—200°C.

9. Method of protecting crops from attack by fungi in which crops subject to or subjected to such attack, seeds of such crops and/or soil in which the said crops are growing or to be grown, are treated with a fungicidally effective amount of one or more compounds of the general formula of claim 1.

**Revendications pour les Etats contractants: CH DE FR GB IT LU**

1. Compositions fongicides comprenant comme constituants possédant une activité fongicide un ou plusieurs composés de la formule générale

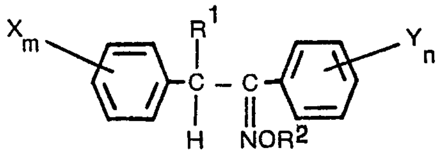

dans laquelle $R^1$ représente un groupe acyloxy, alcoxy, alcoylthio ou arylalcoylthio, $R^2$ représente un atome d'hydrogène, un groupe alcoyle ayant jusqu'à 4 atomes de carbone, un groupe aralcoyle éventuellement substitué ou un groupe acyle, X et Y indépendamment représentent des atomes d'halogènes ou des groupes nitro, alcoyle, alcoxy, alcoxycarbonyle ou méthylènedioxy et m et n sont chacun 0, 1 ou 2, en même temps qu'un véhicule et/ou un agent tensio-actif.

2. Compositions fongicides selon la revendication 1, dans lesquelles $R^1$ représente un groupe méthoxy ou éthoxy.

3. Compositions fongicides selon l'une des revendications 1 et 2, dans lesquelles $R^2$ représente un atome d'hydrogène ou un groupe acyle.

4. Compositions fongicides selon l'une quelconque des revendications 1 à 3, dans lesquelles X et/ou Y représentent un atome d'halogène.

5. Composés possédant une activité fongicide de la formule

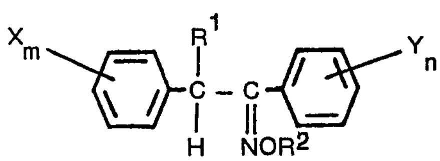

dans laquelle $R^1$, $R^2$, X, Y, m et n ont la signification spécifiée, avec la condition que si $R^1$ représente un groupe alcoxy et $R^2$ est un atome d'hydrogène, alors m et n ne doivent pas être tous deux égaux à zéro.

6. 4-chlorobenzoïne O-méthyléther oxime.

7. 4-méthylbenzoïne O-méthyléther oxime.

8. 4'-chlorobenzoïne O-méthyléther oxime.

9. 4,4'-difluorobenzoïne O-méthyléther oxime.

10. 4-fluorobenzoïne O-méthyléther oxime.

11. Acétyl 4-chlorobenzoïne O-méthyléther oxime.

12. Procédé pour la préparation de composés de la formule générale de la revendication 1, selon lequel des composés de la formule

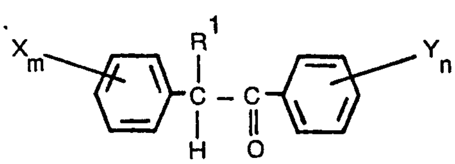

dans laquelle $R^1$ représente un groupe acyloxy ou alcoxy sont mis à réagir avec l'hydroxylamine, après quoi ensuite on fait éventuellement réagir les oximes obtenues avec un composé $R^2Z$, où Z est un

halogène, pour former les dérivés d'oxime correspondants.

13. Procédé selon la revendication 12, dans lequel on utilise des composés de la formule

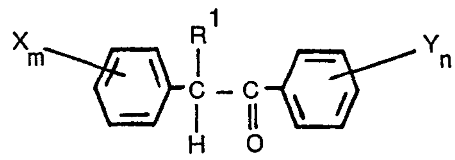

qui ont été obtenus par réaction de composés de la formule

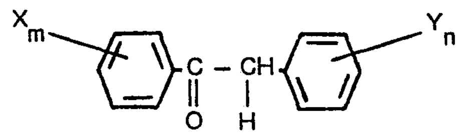

avec du brome en présence d'un solvant sous l'influence d'une irradiation lumineuse, cela étant suivi d'une réaction avec un alcoolate et d'une pyrolyse.

14. Procédé selon la revendication 13, dans lequel la pyrolyse est effectuée à une température comprise entre 160 et 200°C.

15. Méthode de protection de plantes cultivées contre l'attaque par des champignons, selon laquelle des plantes sensibles ou exposées à une telle attaque, des semences de ces plantes et/ou la terre dans laquelle ces plantes poussent ou doivent pousser, sont traitées par une quantité efficace du point de vue fongicide d'un ou plusieurs composés de la formule générale de la revendication 1.

**Revendications pour l'Etat contractant: AT**

1. Compositions fongicides comprenant comme constituants possédant une activité fongicide un ou plusieurs composés de la formule générale

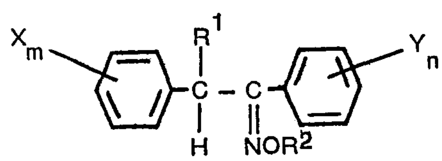

dans laquelle $R^1$ représente un groupe acyloxy, alcoxy, alcoylthio ou aralcoylthio, $R^2$ représente un atome d'hydrogène, un groupe alcoyle ayant jusqu'à 4 atomes de carbone, un groupe aralcoyle éventuellement substitué ou un groupe acyle, X et Y indépendamment représentent des atomes d'halogènes ou des groupes nitro, alcoyle, alcoxycarbonyle ou méthylènedioxy et m et n sont chacun 0, 1 ou 2, en même temps qu'un véhicule et/ou un agent tensio-actif.

2. Compositions fongicides selon la revendication 1, dans lesquelles $R^1$ représente un groupe méthoxy ou éthoxy.

3. Compositions fongicides selon l'une des revendications 1 et 2, dans lesquelles $R^2$ représente un atome d'hydrogène ou un groupe acyle.

4. Compositions fongicides selon l'une quelconque des revendications 1 à 3, dans lesquelles X et/ou Y représentent un atome d'halogène.

5. Compositions fongicides selon la revendication 1, dans lesquelles le constituant possédant une activité fongicide est la 4-chlorobenzoïne O-méthyléther oxime, la 4-méthylbenzoïne O-méthyléther oxime, la 4'-chlorobenzoïne O-méthyléther oxime, la 4,4'-difluorobenzoïne O-méthyléther oxime, la 4-fluorobenzoïne O-méthyléther oxime ou l'acétyl 4-chlorobenzoïne O-méthyléther oxime.

6. Procédé pour la préparation de composés de la formule générale de la revendication 1, selon lequel des composés de la formule

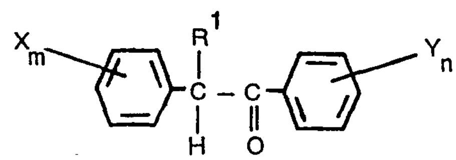

dans laquelle $R^1$ représente un groupe acyloxy ou alcoxy sont mis à réagir avec l'hydroxylamine et ensuite éventuellement on fait réagir les oximes obtenues avec un composé $R^2Z$, où Z est un halogène, de manière à former les dérivés d'oxime correspondants.

7. Procédé selon la revendication 6, dans lequel on utilise des composés de la formule

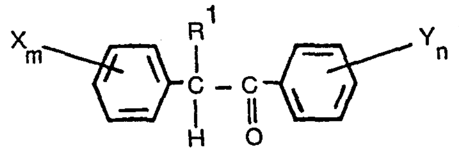

qui ont été obtenus par réaction de composés de la formule

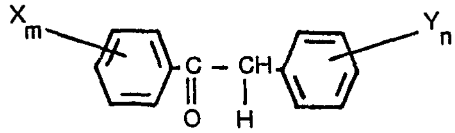

avec le brome en présence d'un solvant sous l'influence d'une irradiation lumineuse, suivie d'une réaction avec un alcoolate et d'une pyrolyse.

8. Procédé selon la revendication 7, dans lequel la pyrolyse est effectuée à une température comprise entre 160 et 200°C.

9. Méthode de protection de plantes cultivées contre l'attaque par des champignons, selon lequel des plantes sensibles ou exposées à une telle attaque, des semences de ces plantes et/ou la terre dans laquelle ces plantes poussent ou doivent pousser sont traitées par une quantité efficace du point de vue fongicide d'un ou plusieurs composés de la formule générale de la revendication 1.

**Patentansprüche für die Vertragsstaaten: CH DE FR GB IT LU**

1. Fungizide Zusammensetzungen, enthaltend als fungizid wirksame Komponenten eine oder mehrere Verbindungen der allgemeinen Formel

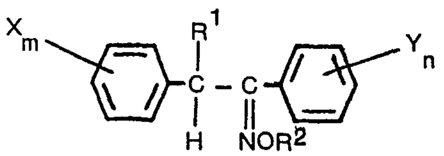

worin $R^1$ eine Acyloxy-, Alkoxy-, Alkylthio- oder Arylalkylthiogruppe darstellt, $R^2$ ein Wasserstoffatom, eine Alkylgruppe mit bis zu 4 Kohlenstoffatomen, eine gegebenenfalls substituierte Aralkyl- oder eine Acylgruppe bedeutet, X und Y unabhängig voneinander Halogenatome, Nitro-, Alkyl-, Alkoxy-, Alkoxycarbonyl- oder Methylendioxygruppen bedeuten und m und n jeweils 0, 1 oder 2 sind; zusammen mit einem Träger und/oder einem oberflächenaktiven Mittel.

2. Fungizide Zusammensetzungen nach Anspruch 1, worin $R^1$ eine Methoxy- oder Ethoxygruppe bedeutet.

3. Fungizide Zusammensetzungen nach Anspruch 1 oder 2, worin $R^2$ ein Wasserstoffatom oder eine Acylgruppe bedeutet.

4. Fungizide Zusammensetzungen nach einem der Ansprüche 1 bis 3, worin X und/oder Y ein Halogenatom bedeuten.

5. Fungizid wirksame Verbindungen der Formel

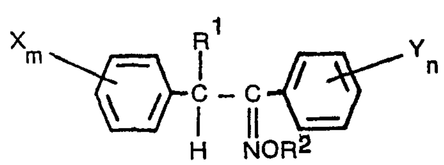

worin $R^1$, $R^2$, X, Y, m und n die oben angegebene Bedeutung mit der Maßgabe haben, daß, falls $R^1$ Alkoxy bedeutet und $R^2$ ein Wasserstoffatom ist, m und n nicht beide gleich 0 sein sollen.

6. 4-Chlorbenzoin-O-methyletheroxim.

7. 4-Methylbenzoin-O-methyletheroxim.

8. 4'-Chlorbenzoin-O-methyletheroxim.

9. 4,4'-Difluorbenzoin-O-methyletheroxim.

10. 4-Fluorbenzoin-O-methyletheroxim.

11. Acetyl-4-chlorbenzoin-O-methyletheroxim.

12. Verfahren zur Herstellung der Verbindungen der allgemeinen Formel des Anspruchs 1, worin Verbindungen der Formel

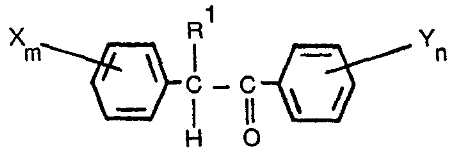

worin $R^1$ eine Acyloxy- oder Alkoxygruppe bedeutet, mit Hydroxylamin umgesetzt werden, worauf gegebenenfalls eine Umsetzung der erhaltenen Oxime mit einer Verbindung $R^2Z$, worin Z Halogen bedeutet, unter Bildung der entsprechenden Oximderivate erfolgt.

13. Verfahren nach Anspruch 12, worin Verbindungen der Formel

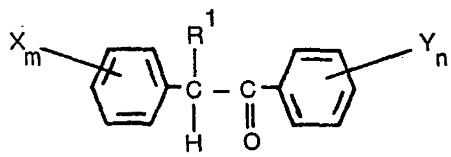

eingesetzt werden, die durch Umsetzung der Verbindungen der Formel

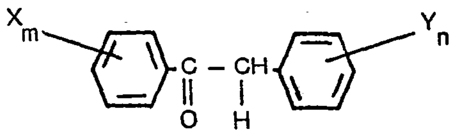

mit Brom in Gegenwart eines Lösungsmittels unter dem Einfluß von Lichtstrahlung, anschließende Umsetzung mit einer Alkoxidverbindung und Pyrolyse erhalten worden sind.

14. Verfahren nach Anspruch 13, worin die Pyrolyse bei einer Temperatur im Bereich von 160—200°C ausgeführt wird.

15. Verfahren zum Schutz von Nutzpflanzen gegen Pilzbefall, bei welchem Nutzpflanzen, die für einen solchen Befall anfällig sind oder diesem unterliegen, Samen solcher Nutzpflanzen und/oder Boden, in welchem diese Nutzpflanzen wachsen oder wachsen sollen, mit einer fungizid wirksamen Menge einer oder mehreren Verbindungen der allgemeinen Formel des Anspruchs 1 behandelt werden.

**Patentansprüche für den Vertragsstaat: AT**

1. Fungizide Zusammensetzungen, enthaltend als fungizid wirksame Komponenten eine oder mehrere Verbindungen der allgemeinen Formel

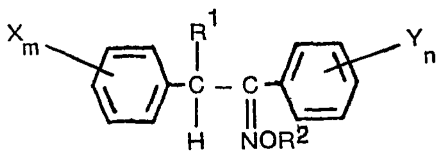

worin $R^1$ eine Acyloxy-, Alkoxy-, Alkylthio- oder Arylalkylthiogruppe darstellt, $R^2$ ein Wasserstoffatom, eine Alkylgruppe mit bis zu 4 Kohlenstoffatomen, eine gegebenenfalls substituierte Aralkyl- oder eine Acylgruppe bedeutet, X und Y unabhängig voneinander Halogenatome, Nitro-, Alkyl-, Alkoxy-, Alkoxycarbonyl- oder Methylendioxygruppen bedeuten und m und n jeweils 0, 1 oder 2 sind; zusammen mit einem Träger und/oder einem oberflächenaktiven Mittel.

2. Fungizide Zusammensetzungen nach Anspruch 1, worin $R^1$ eine Methoxy- oder Ethoxygruppe bedeutet.

3. Fungizide Zusammensetzungen nach Anspruch 1 oder 2, worin $R^2$ ein Wasserstoffatom oder eine Acylgruppe bedeutet.

4. Fungizide Zusammensetzungen nach einem der Ansprüche 1 bis 3, worin X und/oder Y ein Halogenatom bedeuten.

5. Fungizide Zusammensetzung nach Anspruch 1, worin die fungizid wirksame Komponente 4-Chlorbenzoin-O-methyletheroxim; 4-Methylbenzoin-O-methyletheroxim; 4′-Chlorbenzoin-O-methyletheroxim; 4,4′-Difluorbenzoin-O-methyletheroxim; 4-Fluorbenzoin-O-methyletheroxim oder Acetyl-4-chlorbenzoin-O-methyletheroxim ist.

6. Verfahren zur Herstellung der Verbindungen der allgemeinen Formel des Anspruchs 1, worin Verbindungen der Formel

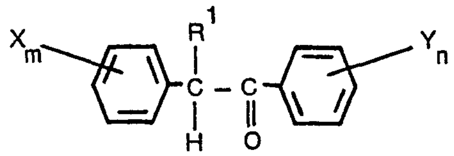

worin $R^1$ eine Acyloxy- oder Alkoxygruppe bedeutet, mit Hydroxylamin umgesetzt werden, worauf gegebenenfalls eine Umsetzung der erhaltenen Oxime mit einer Verbindung $R^2Z$, worin Z Halogen bedeutet, unter Bildung der entsprechenden Oximderivate erfolgt.

7. Verfahren nach Anspruch 6, worin Verbindungen der Formel

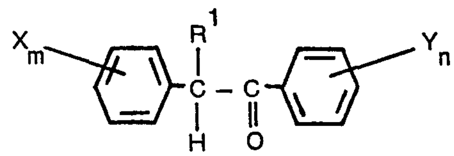

eingesetzt werden, die durch Umsetzung der Verbindungen der Formel

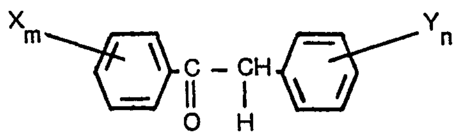

mit Brom in Gegenwart eines Lösungsmittels unter dem Einfluß von Lichtstrahlung, anschließende Umsetzung mit einer Alkoxidverbindung und Pyrolyse erhalten worden sind.

8. Verfahren nach Anspruch 7, worin die Pyrolyse bei einer Temperatur im Bereich von 160—200°C ausgeführt wird.

9. Verfahren zum Schutz von Nutzpflanzen gegen Pilzbefall, bei welchem Nutzpflanzen, die für einen solchen Befall anfällig sind oder diesem unterliegen, Samen solcher Nutzpflanzen und/oder Boden, in welchem diese Nutzpflanzen wachsen oder wachsen sollen, mit einer fungizid wirksamen Menge einer oder mehreren Verbindungen der allgemeinen Formel des Anspruchs 1 behandelt werden.